Europäisches Patentamt

European Patent Office

Office européen des brevets

(11) Veröffentlichungsnummer: **0 210 432**
**A1**

(12) **EUROPÄISCHE PATENTANMELDUNG**

(21) Anmeldenummer: 86108551.2

(22) Anmeldetag: 23.06.86

(51) Int. Cl.⁴: **A 61 M 5/14**

(30) Priorität: 15.07.85 DE 3525191

(43) Veröffentlichungstag der Anmeldung:
04.02.87 Patentblatt 87/6

(84) Benannte Vertragsstaaten:
AT BE CH DE FR GB IT LI LU NL SE

(71) Anmelder: Peter von Berg, Extrakorporale Systeme
-Medizintechnik GmbH
Benzstrasse 2
D-8011 Kirchheim(DE)

(72) Erfinder: von Berg, Peter
Schwabener Weg 1
D-8011 Neukeferloh(DE)

(74) Vertreter: EGLI-EUROPEAN PATENT ATTORNEYS
Widenmayerstrasse 5
D-8000 München 22(DE)

(54) Durchflussregelvorrichtung.

(57) Die Durchflußregelvorrichtung für die Anwendung bei strömenden Flüssigkeiten zur Drucküberwachung bei der Blutdruckmessung hat mindestens einen Durchlaß von einem Einlaß (14) zu einem Auslaß (12). Dieser Durchlaß weist zwei Durchflußpfade auf, nämlich einen nichtsperrbaren, engen Durchflußpfad mit einer Kapillarbohrung (7) und einen von einem "Schnellspülventil" (20) absperrbaren Durchflußpfad mit größerem Öffnungsquerschnitt. Das Schnellspülventil (20) besitzt einen Ventilkörper (21) aus elastisch verformbarem Material, der in einer Kammer (4), die einen Einlaß (5) und einen Auslaß (9) besitzt, angeordnet ist und durch einen Stößel (28) gedehnt werden kann, wobei sich sein Querschnitt verringert. Im Ruhezustand leigt die Aussenseite des Ventilkörpers an den Wänden der Kammer (4) an und sperrt den Durchflußpfad. Wird der Stößel niedergedrückt, so verformt sich der Ventilkörper (21) elastisch, verringert dabei seinen Querschnitt und gibt den Durchflußpfad rings um den Ventilkörper (21) herum vom Einlaß (5) zum Auslaß (9) frei.

FIG.1

Croydon Printing Company Ltd.

EP 0 210 432 A1

Durchflußregelvorrichtung

Beschreibung

Die Erfindung bezieht sich auf eine Durchflußregelvorrichtung für die Anwendung bei strömenden Flüssigkeiten zur Drucküberwachung bei der Blutdruckmessung gemäß dem Oberbegriff des Patentanspruches 1.

Eine derartige Durchflußregelvorrichtung ist aus der DE-PS 30 23 435 bzw. der US-PS 4 341 224 bekannt.

Generell muß bei der kontinuierlichen intravasalen Druckmessung verhindert werden, daß sich an der Kanülenspitze Blutkoagulationen bilden. Daher ist eine kontinuierliche Spülung notwendig. Mit Hilfe eines unter Druck stehenden Spülflüssigkeitsbehälters und der Durchflußregelvorrichtung wird die Kanüle offengehalten. Die Menge der normalerweise fließenden Spülflüssigkeit wird durch die Kapillarbohrung bestimmt. Übliche Durchflußraten liegen in der Größenordnung von 3 - 6 ml/h.

Um den Patienten nicht zu gefährden und um das Meßergebnis nicht zu verfälschen, muß das gesamte Meßsystem absolut frei von Luftblasen sein; es muß also vor Inbetriebnahme bzw. vor Anschluß an den Patienten entlüftet werden. Hierzu ist der sperrbare Durchlaß für die Spülflüssigkeit vorgesehen, der einen erheblich größeren Öffnungsquerschnitt hat als die Kapillarbohrung. Solange dieser sperrbare Durchlaß geöffnet ist, strömt die Spülflüssigkeit durch den großen Öffnungsquerschnitt und füllt somit innerhalb kurzer Zeit das gesamte System. Nach dem Sperren dieses Durchlasses gelangt dagegen nur die durch die Kapillarbohrung begrenzte Spülflüssigkeitsmenge in das System. Bei der Durchflußregelvorrichtung der DE-PS 30 23 435 ist der Ventilkörper mittels eines Dichtungsringes (O-Ring) in der Ruhestellung gegenüber dem Ventilsitz am Gehäuse abgedichtet. Die Kapillarbohrung befindet sich in einem Einsatz innerhalb des Ventilkörpers, wobei der Ventilkörper stromaufwärts der Kapillarbohrung einen Durchlaß aufweist, der mit dem Einlaß der Vorrichtung in Strömungsverbindung steht. Der Ventilkörper wird durch eine separate Feder in die geschlossene Stellung gedrückt. Zum Öffnen wird der Ventilkörper gegen die Kraft dieser Feder verschoben.

Eine ähnliche Durchflußregelvorrichtung, bei der der Ventilkörper in gleicher Weise gegenüber dem Ventilsitz bzw. dem Gehäuse angeordnet ist, ist der US-PS 4 497 468 und der US-PS 4 291 702 zu entnehmen.

Schließlich zeigt die US-PS 3 675 891 eine ähnliche Durchflußregelvorrichtung, bei welcher der absperrbare Durchlaß mit größerem Querschnitt parallel, d.h. als Bypass zur Kapillarbohrung liegt. Auch hier ist der Ventilkörper federnd gegen den Ventilsitz im Gehäuse gedrückt. Zum Öffnen des Durchlasses für die Schnellspülung muß auch hier der Ventilkörper gegen die Kraft dieser Feder vom Ventilsitz abgehoben werden.

Generell ist noch anzumerken, daß die bekannten Durch- flußregelvorrichtungen und die Durchflußregelvorrichtun- gen nach der Erfindung generell nur für den einmaligen Gebrauch bestimmt sind. Sie müssen daher möglichst kostengünstig hergestellt werden können und trotzdem einwandfrei funktionieren, was insbesondere hohe Anforderungen an die Dichtigkeit des Ventilsitzes des absperrbaren Durchlasses stellt.

Diese Forderungen sind bei dem beschriebenen Stand der Technik noch nicht zufriedenstellend gelöst. Vielmehr bestehen die bekannten Vorrichtungen aus vielen, teilweise kompliziert herzustellenden und aufwendig zu montierenden Einzelteilen, so daß Herstellungs- und Montageaufwand für eine nur für den einmaligen Gebrauch bestimmte Vorrichtung noch zu hoch sind.

- 4 -

Aufgabe der Erfindung ist es, die Durchflußregelvorrichtung der eingangs genannten Art dahingehend zu verbessern, daß sie einfacher aufgebaut ist, gleichwohl aber einwandfrei und sicher funktioniert.

Diese Aufgabe wird durch die im Kennzeichenteil des Patentanspruches 1 angegebenen Merkmale gelöst. Vorteilhafte Ausgestaltung und Weiterbildungen der Erfindungen sind den Unteransprüchen zu entnehmen.

Der Grundgedanke der Erfindung besteht darin, den Ventilkörper aus elastischem Material so auszubilden, daß er sich bei Betätigen des "Schnellspülventiles" so streckt und dabei verformt, daß der gewünschte Öffnungsquerschnitt entsteht. Gleichzeitig wird die Federeigenschaft dieses Ventilkörpers ausgenutzt, das Ventil in der geschlossenen Ruhestellung zu halten, so daß keine zusätzliche Feder benötigt wird. Ein weiterer Vorteil der Erfindung liegt darin, daß - im Gegensatz zu den bekannten Vorrichtungen - keine hohen Anforderungen an die Oberflächengüte des "Ventilsitzes" gestellt werden müssen, da der "Ventilsitz" sehr großflächig ausgebildet ist und Unebenheiten durch elastische Materialverformung des Ventilkörpers ohne weiteres ausgeglichen werden.

Im folgenden wird die Erfindung anhand eines Ausführungsbeispiels im Zusammenhang mit der Zeichnung ausführlicher erläutert. Es zeigt:

Fig. 1    eine Schnittansicht der Durchflußregelvorrichtung nach der Erfindung bei geschlossenem Schnellspüldurchlaß (Ruhestellung); und

Fig. 2      eine Schnitansicht ähnlich Fig. 1 jedoch mit geöffnetem Schnellspüldurchlaß (Betätigungsstellung).

Die Durchflußregelvorrichtung besitzt eine Gehäuse 1, das durch einen Deckel 2 verschlossen ist. Im Inneren des Gehäuses 1 sind zwei Kammern 3 und 4 vorgesehen, die im wesentlichen zylindrisch sind und parallel zueinander liegen. Die beiden Kammern 3 und 4 stehen über einen Durchlaß 5 im Bereich ihrer oberen Enden miteinander in Verbindung. Die Kammer 3 ist für den engen, nicht-sperrbaren Durchflußpfad vorgesehen, während die Kammer 4 für den absperrbaren Schnell- spüldurchlaß vorgesehen ist. In der Kammer 3 befindet sich ein Glasröhrchen 6, das eine Kapillarbohrung 7 mit sehr engem Öffnungsquerschnitt aufweist. Beide Kammern 3 und 4 haben an ihrem unteren Ende eine Öffnung 8 bzw. 9, die gemeinsam in einen Kanal 10 münden. Dieser Kanal läuft quer zur Hauptrichtung der beiden Kammern 3 und 4 und endet beidseitig in Anschlußstücken 11 bzw. 12, die einstückig an dem Gehäuse angeformt sind. An das Anschlußstück 11 wird eine nichtdargestellte Druckmeßdose angeschlossen, die über eine Membran flüssigkeitsdicht gegenüber dem Kanal 10 abgesperrt ist. An das Anschlußstück 12 (Auslass) wird ein (nicht-dargestellter) Schlauch angeschlossen, beispielsweise aufgesteckt, der zum Patienten führt und an seinem freien Ende eine Kanülenspitze trägt.

Der Deckel 2 besitzt gegenüberliegend zur Kammer 3 einen zylindrischen Vorsprung 13, der in das obere Ende der Kammer 3 hineinragt. Dieser Vorsprung besitzt eine sich vollständig durch den Deckel hindurch erstreckende Durchgangsbohrung 14, die mit der Kapillarbohrung 7 ausgefluchtet ist. Diese Durchgangsbohrung 14 mündet an der Außenseite des Deckels 2 in ein mit dem Deckel 2 einstückig ausgebildetes Anschlußstück 15 (Einlass), auf das ein Schlauch 35 aufsteckbar ist, der mit einem (nicht-dargestellten Behälter für die Spülflüssigkeit verbunden ist, ggf. unter Zwischenschaltung einer Tropfkammer.

Der Vorsprung 13 besitzt eine quer zur Durchgangsbohrung 14 verlaufende und an diese angeschlossene Öffnung 16, die mit der Öffnung 5 in einer die beiden Kammern 3 und 4 trennenden Wand 17 ausgefluchtet ist.

Das Glasröhrchen 6 ist mittels eines Dichtungsringes 18 gegenüber der Unterseite des Vorsprunges 13 abgedichtet, so daß Flüssigkeit aus der Durchgangsbohrung 14 nur in die Kapillarbohrung 7 eintreten kann, nicht jedoch in den sonstigen Hohlraum der Kammer 3. In ähnlicher Weise ist das untere Ende des Glasröhrchens 6 mittels eines Dichtungsringes 19 am unteren Ende der Kammer 3 gehalten, so daß Flüssigkeit aus der Kapillarbohrung 7 nur durch die Öffnung 8 abfließen kann.

In der Kammer 4 befindet sich ein absperrbares Schnellspülventil 20, dessen Ventilkörper 21 aus elastischem Material wie z.B. Silikonkautschuk oder Gummi hergestellt ist. Dieser Ventilkörper 21 ist im wesentlichen zylindrisch ausgebildet, besitzt an seinem oberen Ende einen radial nach außen abstehenden Wulst 22 und eine zylindrische Ausnehmung 23, die in Richtung zum Wulst 22 offen ist. An der dem Wulst 22 abgewandten Unterseite läuft der Ventilkörper konisch spitz zu in Form eines an seiner Spitze abgerundeten Kegelstumpfes 24.

Der Ventilkörper 21 ist in die Kammer 4 eingesetzt und liegt mit der nach unten weisenden Kante des Wulstes 22 an einer umlaufenden Stufe 25 der Kammer 4 auf.

Gegenüberliegend zu dem Wulst 22 besitzt der Deckel 2 einen zylindrischen, umlaufenden Vorsprung 26, der in die Kammer 4 hinreinragt und auf der Oberseite des Wulstes 22 aufliegt. Der Wulst 22 ist also zwischen der Stufe 25 und dem Vorsprung 26 eingespannt und damit fest in der Kammer 4 gehalten. Gleichzeitig dient der Wulst 22 als Abdichtung zwischen dem Gehäuse 1 und dem Deckel 2.

Der Deckel 2 weist eine mit dem zylindrischen Vorsprung 26 und dem Ventilkörper 21 ausgefluchtete Öffnung 27 auf, durch welche ein Ventilbetätigungsorgan hindurchragt. Dieses Ventilbetätigungsorgan besteht einstückig aus einem zylindrischen Stößel 28, einem Schaft 29 und einem Betätigungsknopf 30. Der Schaft 29 ist in der Öffnung 27 geführt und besitzt im Übergangsbereich zum Stößel 28 einen Schnappring 31 in Form eines radial vorstehenden umlaufenden Bundes, dessen radial nach außen weisende Seite mit einer Fase versehen ist, die in Richtung zum Stößel 28 spitz zuläuft.

An seinem unteren, freien Ende ist der Ventilkörper 28 kegelstumpfförmig konisch ausgebildet, wobei die Kegelstumpfspitze 32 abgerundet ist.

Der Stößel wird von der Außenseite des Deckels 2 in die Öffnung 27 eingeführt, bis der Schnappring 21 hinter der Kante der Öffnung 27 eingerastet ist. Die zylindrische Öffnung des Vorsprunges 26 hat dabei einen etwas größeren Durchmesser als die Öffnung 27. Damit ist das Betätigungsorgan in axialer Richtung gegen Herausziehen aus dem Deckel gesichert. Der an dem Schaft 29 angeformte Betätigungsknopf 30 hat einen größeren Durchmesser als der Schaft 29, womit ein axialer Begrenzungsanschlag gegen ein Hineindrücken des Betätigungsorganes gebildet ist. Der Stößel 28 hat einen Durchmesser, der dem Durchmesser der zylindrischen Ausnehmung 23 des Ventilkörpers 21 entspricht, wobei sich im Ruhezustand im Bereich des Kegelstumpfes 32 ein Hohlraum ergibt. Die Außenwandung des Ventilkörpers liegt im Ruhezustand über einen weiten Bereich an der Innenwandung der Kammer 4 an, so daß der Durchlaß 5 zwischen den Kammern 3 und 4 abgedichtet ist. Unmittelbar anschließend an den Durchlaß 5 besitzt die die Kammer 3 und 4 trennende Wand 17 eine zur Kammer 4 hinweisende Abschrägung 33, so daß Flüssigkeit ungehindert durch den Durchlaß 5 an dem Vorsprung 26 und dem Wulst 22 vorbeifließen kann.

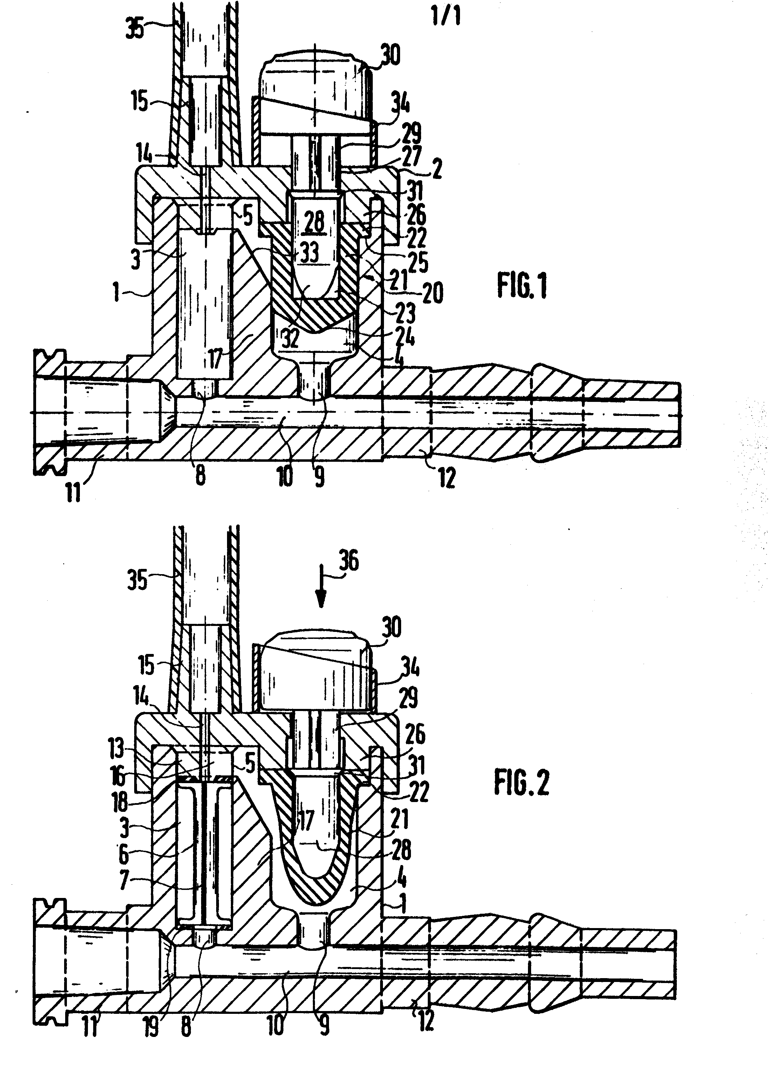

Im Ruhezustand zieht sich der Ventilkörper 21 aufgrund der federelastischen Eigenschaften seines Materiales zu seiner Grundform zusammen, so daß der Stößel bzw. das gesamte Betätigungsorgan so weit in Richtung zum Deckel 2 gedrückt ist, daß der Schnappring 31 am Deckel zum Anschlag kommt. In dieser Stellung ist die Wand des Ventilkörpers 21 relativ dick, so daß sie an der Innenwandung der Kammer 4 anliegt. Damit ist der Durchlaß 5 (Einlaß zur Kammer 4) abgesperrt. Wird nun der Betätigungsknopf 30 in Richtung des Pfeiles 36 niedergedrückt, so wird der Ventilkörper 21 durch den Stößel 28 gestreckt und verformt sich dabei so, daß die Wandung des Ventilkörpers 21 dünner wird. Dies ist in Fig. 2 dargestellt. Die Innenseite des Ventilkörpers schmiegt sich dabei rings an den Außenumfang des Stößels 28 an, während die Außenseite des Ventilkörpers 21 von der Innenwand der Kammer 4 abhebt und damit einen Durchgangsweg von dem Durchlaß 5 durch die Kammer 4 hindurch zu der Öffnung 9 freigibt. Spülflüssigkeit aus dem (nicht dargestellten) Behälter strömt damit aus dem Schlauch 35 durch die Durchgangsbohrung 14 hindurch über die Öffnung 16 zum Durchlaß 5, durch die Kammer 4 hindurch zur Öffnung 9 und damit in den Kanal 10. Aufgrund des relativ großen Öffnungsquerschnittes dieses Strömungsweges wird eine "Schnellspülung" erreicht, so daß die ganze Durchflußregelvorrichtung schnell entlüftet werden kann.

Wird der Druckknopf 30 aus der Stellung der Fig. 2 wieder losgelassen, so drückt der Ventilkörper aufgrund seiner federelastischen Eigenschaften den Stößel und damit das gesamte Betätigungsorgan wieder in die Ruhestellung der Fig. 1 zurück.

An dem Deckel 2 ist noch eine koaxial zu dem Betätigungsknopf 30 angeordnete Hülse 34 befestigt, die den Druckknopf 30 teilweise umgibt und an ihrem oberen Ende abgeschrägt ist. Diese Hülse 34 dient als Schutz gegen eine unbeabsichtigte Betätigung des Betätigungsknopfes 30.

Kurz zusammengefaßt werden mit der Ausgestaltung des Ventilkörpers und des Betätigungsorganes folgende Vorteile erreicht:

- die gesamte Durchflußregelvorrichtung benötigt weniger Einzelteile und insbesondere auch keine separate Feder, die das Betätigungsorgan in ihre Ruhestellung drückt;

- der Ventilkörper dient gleichzeitig als Dichtungselement zum Absperren des Durchlasses, als Rückholfeder und als Dichtungselement zur Abdichtung des Gehäuses 1 gegenüber dem Deckel 2.

Durch den Kegelstumpf 24 am unteren, freien Ende des Ventilkörpers 21 wird in diesem Bereich eine Materialanhäufung geschaffen. Diese Materialanhäufung stellt zum einen sicher, daß der Ventilkörper bei Niederdrücken des Stößels in seinem unteren Bereich nur geringfügig verformt wird, so daß die gewünschte Einschnürung an der Seitenwand des Ventilkörpers erfolgt. Weiterhin dient sie dazu, das Füllvolumen der Kammer 4 zu verringern, so daß die Entlüftungszeit verkürzt wird.

Abschliessend sei noch erwähnt, daß die Erfindung nicht auf das dargestellte Ausführungsbeispiel beschränkt ist. So kann beispielsweise die Kapillarbohrung auch hier ähnlich wie bei der US-PS 4 201 702 koaxial durch den Stößel 28 hindurch verlaufen, wobei dann der Ventilkörper an seinem unteren Ende eine Durchgangsöffnung haben muß. Damit sich diese in der Ruhestellung nicht verschließt, kann der Stößel 28 beispielsweise einen zylindrischen Ansatz mit Kapillarbohrung haben, der durch eine Bohrung in der Spitze des Ventilkörpers hindurchgesteckt ist.

Sämtliche in den Patentansprüchen, der Beschreibung und der Zeichnung dargestellten technischen Einzelheiten können sowohl für sich als auch in beliebiger Kombination miteinander erfindungswesentlich sein.

## Durchflußregelvorrichtung

-----------------------------

## Patentansprüche

1. Durchflußregelvorrichtung für die Anwendung bei strömenden Flüssigkeiten zur Drucküberwachung bei der Blutdruckmessung, mit einem Gehäuse mit zumindest einem Durchlaß, der einen Einlaß und einen Auslaß aufweist, wobei an den Auslaß ein bei Gebrauch fortlaufend durchströmter Katheter anschließbar ist,

- 12 -

mit einer einen engen, nicht-sperrbaren Durchflußpfad vom Einlaß zum Auslaß gewährleistenden Kapillarbohrung und mit einem im Durchlaß angeordneten Ventil zum Öffnen und Absperren eines weiteren Durchflußpfades mit größerem Querschnitt vom Einlaß zum Auslaß rings um einen Ventilkörper des Ventiles, wobei dieses Ventil durch Federkraft in einer den weiteren Durchflußpfad sperrenden Ruhestellung gehalten ist, dadurch gekennzeichnet, daß der Ventilkörper (21) aus elastisch verformbaren Material ist und so gestaltet ist, daß er sich bei Verschieben eines mit dem Ventilkörper in Wirkverbindung stehenden Stößels (28) unter Verringerung seines Querschnittes dehnt und damit das Ventil öffnet.

2. Durchflußregelvorrichtung nach Anspruch 1, dadurch gekennzeichnet, daß der Ventilkörper (28) zylin-drisch ausgebildet ist, eine innere, zum Stößel (21) hin, offene zylindrische Ausnehmung (23) und einen im Bereich der Öffnung der Ausnehmung (23) radial nach außen vorstehenden Wulst (22) aufweist, der den Ventilkörper (21) gegenüber dem Gehäuse (1) hält, wobei der Stößel (28) in die Ausnehmung (23) hineinragt.

3. Durchflußregelvorrichtung nach Anspruch 1 oder 2, dadurch gekennzeichnet, daß das in die Ausnehmung (23) des Ventilkörpers (21) hineinragende Ende des Stößels (28) die Form eines an seiner Spitze abgerundeten Kegelstumpfes (32) hat.

4. Durchflußregelvorrichtung nach einem der Ansprüche 1 bis 3, dadurch gekennzeichnet, daß der Durchmesser des zylindrischen Teiles des Stößels (28) gleich dem Innendurchmesser der zylindrischen Ausnehmung (23) des Ventilkörpers (21) ist.

5. Durchflußregelvorrichtung nach einem der Ansprüche 1 bis 4, dadurch gekennzeichnet, daß der Ventilkörper (21) mittels des Wulstes (22) zwischen einer Stufe (25) des Gehäuses und (1) einem das Gehäuse verschliessenden Deckel (2) eingespannt ist.

6. Durchflußregelvorrichtung nach einem der Ansprüche 1 bis 5, dadurch gekennzeichnet, daß das freie, verschiebliche Ende des Ventilkörpers (21) die Form eines Kegelstumpfes (24) hat, so daß in diesem Bereich eine Materialanhäufung vorhanden ist.

7. Durchflußregelvorrichtung nach einem oder mehreren der Ansprüche 1 - 6, dadurch gekennzeichnet, daß der Ventilkörper (21) in einer zylindrischen Kammer (4) angeordnet ist, welche einen Einlaß (5) und einen Auslaß (6) aufweist, wobei der Innendurchmesser der Kammer (4) dem Außendurchmesser des Ventilkörpers (21) in dessen Ruhelage entspricht, so daß der Ventilkörper (21) in seiner Ruhestellung den Durchflußpfad zwischen dem Einlaß (5) und dem Auslaß (9) sperrt.

8. Durchflußregelvorrichtung nach einem der Ansprüche 1 bis 7, dadurch gekennzeichnet, daß der Deckel (2) eine Öffnung (27) aufweist, durch welche ein Schaft (29) des Stößels (28) hindurchragt und daß im Übergangsbereich zwischen dem Schaft (29) und dem Stößel (28) ein mit einer Fase versehener Schnapp-

- 14 -

ring (21) angeformt ist, welcher hinter der Kante der Öffnung (27) einrastet.

9. Durchflußregelvorrichtung nach einem der Ansprüche 1 bis 8, dadurch gekennzeichnet, daß ein an den Einlaß (5) der Kammer (4) angrenzender Bereich einer Wand (17) der Kammer (4) eine Abschrägung (33) aufweist.

10. Durchflußregelvorrichtung nach einem der Ansprüche 1 bis 9, dadurch gekennzeichnet, daß der Ventilkörper (21) aus Silikonkautschuk ist.

11. Durchflußregelvorrichtung nach einem der Ansprüche 1 bis 10, dadurch gekennzeichnet, daß der Stößel (28), der Schaft (29), der angeformte Schnappring (31) und ein am anderen Ende des Schaftes (29) angebrachter Betätigungsknopf (30) einstückig aus Kunststoff geformt sind.

0210432

1/1

FIG.1

FIG.2

0210432

Europäisches Patentamt

**EUROPÄISCHER RECHERCHENBERICHT**

Nummer der Anmeldung

EP 86 10 8551

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-2 112 208 (LE VOY'S INC.) <br> * Seite 6, Zeile 32 - Seite 7, Zeile 22; Figuren 2,5 * | 1,2,11 | A 61 M 5/14 <br> A 61 M 25/00 <br> F 16 K 7/02 |
| | --- | | |
| A | DE-B-1 142 087 (RENAULT) <br> * Figur 4 * | 3,9 | |
| | --- | | |
| A | US-A-2 649 276 (T. TITUS) <br> * Figur 1 * | 4-6 | |
| | --- | | |
| A | US-A-3 768 514 (GOTO) <br> * Spalte 4, Zeilen 24-46; Figuren 1,2 * | 7 | |
| | --- | | |
| A | US-A-4 090 503 (SPEIDEL) <br> * Figuren 2,3 * | 1 | RECHERCHIERTE SACHGEBIETE (Int. Cl.4) |
| | --- | | |
| A,D | GB-A-2 054 802 (GOULD INC.) <br> * Figuren * | 1 | A 61 M <br> F 16 K <br> A 61 B |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 27-11-1986 | EHRSAM F.J.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82